# FASCICULE DE BREVET EUROPEEN

(11) **EP 2 783 623 B1**
(45) Date de publication et mention de la délivrance du brevet: **01.08.2018**
(21) Numéro de dépôt: 14161174.9
(22) Date de dépôt: 21.03.2014
(51) Int. Cl.: A61B 3/024, A61B 3/00, A61B 3/032

(54) **Dispositif de détermination et/ou d'analyse du champ visuel d'un patient**
Vorrichtung zur Bestimmung und/oder zur Analyse des Sehfeldes eines Patienten
Device for determining and/or analysing the visual field of a patient

(30) Priorité: 26.03.2013 FR 1352689
(43) Date de publication de la demande: 01.10.2014
(73) Titulaire: FIM Medical, 69100 Villeurbanne (FR)
(72) Inventeur: Hannes, Benjamin, 69630 Chaponost (FR)
(74) Mandataire: Verriest, Philippe

(56) Documents cités:
- US-A- 3 891 311
- US-A- 5 035 500
- US-A- 5 459 536
- US-A1- 2008 024 726

## Description

La présente invention concerne un dispositif de détermination et/ou d'analyse du champ visuel d'un patient. La présente invention concerne également un appareil d'exploration de la fonction visuelle comprenant le dispositif.

Le champ visuel d'un patient est déterminé par un examen réalisé à l'aide d'un appareil de mesure dédié. Il est connu d'utiliser un appareil de mesure présentant une coupole hémisphérique concave débouchant en regard d'un emplacement prévu pour le visage du patient.

La tête du patient est maintenue en position par une mentonnière et un appui-tête pour que les yeux du patient se trouvent au centre de la coupole hémisphérique en regard du pôle de la coupole.

Un point de repère lumineux, généré par l'appareil au pôle, doit être fixé par le patient pour que le regard du patient reste droit durant l'examen.

L'examen consiste pour l'appareil à allumer successivement plusieurs sources lumineuses ponctuelles à différents emplacements de la coupole concave. Le patient signale, en appuyant sur un bouton de l'appareil, chaque source lumineuse qu'il perçoit.

Après avoir soumis au patient un ensemble de sources lumineuses réparties sur la surface concave de la coupole, l'appareil détermine les points signalés et en déduit le champ visuel du patient.

Cet appareil de mesure donne satisfaction en ce que la détermination du champ visuel du patient est fiable et peut être réalisée en une seule fois. Des dispositifs typiques de mesure du champ visuel sont décrits, par exemple, dans les documents US 5 035 500 et US 3 891 311. Toutefois, la détermination du champ visuel n'est généralement pas le seul examen oculaire réalisé sur un patient, ce qui oblige le patient à se déplacer entre l'appareil de mesure dédié et d'autres appareils d'examens oculaires.

Cette situation peut être une source de perte de temps, notamment lorsque des examens oculaires doivent être effectués sur un ensemble de patients dans le but de dépister des affections oculaires. De plus, la présence d'une multitude d'appareils d'examens oculaires nécessite un espace conséquent.

La présente invention vise à résoudre tout ou partie des inconvénients mentionnés ci-dessus.

A cet effet, la présente invention concerne un dispositif de détermination et/ou d'analyse du champ visuel d'un patient, comprenant une partie fixe destinée à être solidarisée à un appareil d'exploration de la fonction visuelle du patient, l'appareil comprenant un emplacement de positionnement apte à recevoir le visage du patient.

Le dispositif comprend aussi une partie mobile pourvue de deux secteurs comprenant chacun une pluralité de sources lumineuses ponctuelles agencées pour s'allumer et s'éteindre, la partie mobile étant déplaçable entre une position déployée dans laquelle chaque secteur est situé en regard d'un oeil du patient lorsque la partie fixe est solidarisée à l'appareil et le visage du patient est disposé dans l'emplacement de positionnement, et une position escamotée.

Le dispositif comprend également des moyens de déplacement de la partie mobile entre la position escamotée et la position déployée.

Le dispositif est intégré dans un appareil d'exploration de la fonction visuel réalisant une batterie d'examen de la vision du patient.

Ainsi l'appareil est apte à réaliser la batterie d'examens de la vision lorsque la partie mobile est en position escamotée et l'examen du champ visuel lorsque la partie mobile est en position déployée.

La batterie d'examens de la vision et l'examen du champ visuel sont réalisés lorsque le visage du patient est situé dans l'emplacement de positionnement de l'appareil.

Selon un aspect de l'invention, les moyens de déplacement sont agencés pour déplacer en translation la partie mobile entre ses positions escamotée et déployée.

Selon un aspect de l'invention, la partie mobile est sensiblement plane et s'étend selon un plan d'extension. Selon un aspect de l'invention, les moyens de déplacement sont agencés pour déplacer en translation la partie mobile parallèlement au plan d'extension de cette dernière.

Selon un mode de réalisation préféré, les secteurs sont plans et appartiennent au plan d'extension.

Selon un aspect de l'invention, les moyens de déplacement de la partie mobile comprennent un pignon monté sur la partie fixe et une crémaillère prévue sur la partie mobile, le pignon coopérant avec la crémaillère de manière à ce que la rotation du pignon provoque le déplacement de la partie mobile.

Selon un aspect de l'invention, la partie fixe comprend une unité d'entrainement et de commande de la rotation du pignon.

Selon un aspect de l'invention, l'unité d'entrainement et de commande comprend un moteur électrique. Selon un autre aspect de l'invention, l'unité d'entrainement et de commande comprend une carte électronique de commande de la rotation du moteur.

Selon un aspect de l'invention, la partie mobile comprend des moyens de commande de l'allumage et de l'extinction des sources lumineuses des secteurs.

Les moyens de commandes sont agencés pour permettre l'activation des sources lumineuses des secteurs dans un ordre aléatoire, déterminé au préalable ou par une commande externe en communication avec les moyens de commandes.

Selon un aspect de l'invention, les moyens de commande comprennent une carte électronique montée sur la partie mobile. Selon un mode de réalisation préféré, les sources lumineuses des secteurs sont montées sur la carte électronique.

Selon un aspect de l'invention, chaque secteur comprend un repère visuel autour duquel sont réparties les sources lumineuses dudit secteur.

Les repères visuels sont agencés pour permettre au patient d'orienter, en conditions d'utilisation, les centres optiques des yeux selon un axe optique.

Les sources lumineuses des secteurs sont ainsi disposées de façon décalée par rapport aux axes optiques des yeux du patient, lorsque le patient regarde en direction des repères.

Selon un aspect de l'invention, chaque repère visuel est formé par une ouverture ménagée dans la partie mobile.

Selon un aspect de l'invention, chaque ouverture est de forme rectangulaire et débouchante dans le sens de la direction donnée.

La présente invention concerne également un appareil d'exploration de la fonction visuelle comprenant un emplacement de positionnement apte à recevoir le visage d'un patient, et un dispositif.

Selon un aspect de l'invention, le dispositif étant solidaire de l'appareil. Par solidaire, nous entendons que le dispositif est rapporté sur l'appareil ou que le dispositif et l'appareil sont réalisés en une pièce.

Selon un aspect de l'invention, l'appareil d'exploration est agencé pour que les centres optiques des yeux du patient soient situés dans un plan horizontal lorsque le visage du patient est dans l'emplacement de positionnement. Selon un aspect de l'invention, l'appareil d'exploration est agencé pour que les axes optiques appartiennent au plan horizontal.

Selon un aspect de l'invention, les sources lumineuses de chaque secteur sont réparties autour du repère visuel dudit secteur de telle sorte que, en position déployée de la partie mobile, les sources lumineuses présentent par rapport au centre optique et à l'axe optique correspondant audit secteur des angles d'incidence inférieurs à 45°. De préférence, les angles d'incidence sont inférieurs à 40°, et en particulier inférieur à 30°.

Selon un aspect de l'invention, les secteurs sont symétriques par rapport à un plan vertical passant par le segment de l'emplacement de positionnement destiné à recevoir l'arête du nez du patient.

Selon un aspect de l'invention, le plan d'extension de la partie mobile est sensiblement vertical et est situé entre les yeux et la pointe du nez du patient lorsque le visage du patient est disposé dans l'emplacement de positionnement.

Selon un aspect de l'invention, la partie mobile présente une encoche destinée à laisser apparaitre dans le champ de vision du patient, une zone centrale de la paroi interne de l'appareil d'exploration. La zone centrale comprend des diodes électroluminescentes.

Selon un aspect de l'invention, le plan d'extension de la partie mobile est orthogonal au plan vertical.

Selon un aspect de l'invention, l'appareil comprend des sources lumineuses supplémentaires montées sur une paroi interne de l'appareil, afin que chaque source lumineuses supplémentaire présente par rapport au centre optique et à l'axe optique de l'oeil le plus proche de ladite source:
- un angle d'incidence entre -30° et +30° par rapport à l'horizontale, le sens positif des angles étant défini vers le haut et
- un angle d'incidence entre +30° et +90° par rapport à la verticale, le sens positif des angles étant défini vers le champ visuel temporal de l'oeil le plus proche.

Selon un aspect de l'invention, les sources lumineuses des secteurs et les sources lumineuses supplémentaires comprennent des diodes électroluminescentes.

De toute façon l'invention sera bien comprise à l'aide de la description qui suit en référence aux dessins schématiques annexés représentant, à titre d'exemple non limitatif, une forme d'exécution de dispositif.
Figure 1 est une vue de face de l'appareil d'exploration de la fonction visuelle comprenant un dispositif de détermination et/ou d'analyse du champ visuel d'un patient, une partie mobile du dispositif étant en position escamotée.
Figure 2 est une vue de face de l'appareil d'exploration comprenant le dispositif, la partie mobile étant en position déployée.
Figure 3 est une vue de devant en perspective du dispositif comprenant une partie mobile et une partie fixe.
Figure 4 est une vue arrière en perspective du dispositif comprenant la partie mobile et la partie fixe.
Figure 5 est une vue arrière en perspective de la partie mobile.
Figure 6 est une vue de devant en perspective d'un circuit électronique monté dans la partie mobile.

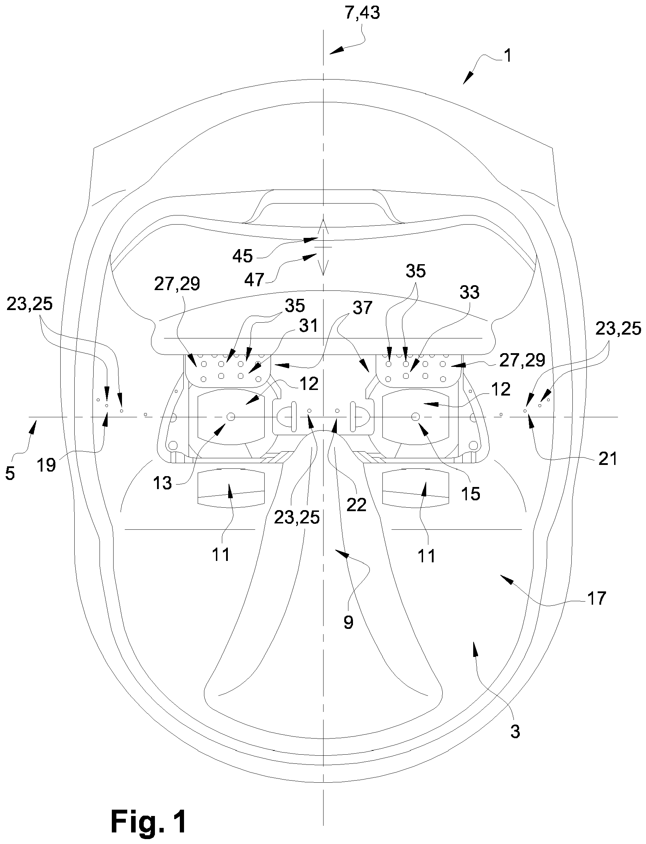

Comme illustré à la figure 1, un appareil 1 d'exploration de la fonction visuelle comprend un emplacement 3 de positionnement du visage d'un patient. Le patient est considéré dans le présent texte comme un utilisateur de l'appareil de la fonction visuelle.

Pour toute la description qui suit, il est considéré que le visage du patient est situé dans l'emplacement 3 de positionnement et que l'appareil 1 est agencé pour que les centres optiques des yeux du patient appartiennent à un plan horizontal 5 et que le plan passant par l'arête du nez du patient est appelé le plan vertical 7.

L'emplacement 3 comprend des lucarnes 11 alignées verticalement avec les yeux du patient et étant distantes du plan horizontal 5 en direction du bas du visage du patient. Les lucarnes 11, agencés pour réaliser des tests de la vision de près, ne sont pas décrites plus en détail dans le présent texte.

L'emplacement 3 comprend une cavité 9 de positionnement pour le nez du patient et deux fenêtres 12 d'observation de forme sensiblement plane et rectangulaire en regard des yeux du patient.

Les fenêtres 12 sont agencées pour permettre au patient de d'orienter le centre optique de l'oeil gauche et le centre optique de l'oeil droit selon un axe optique gauche 13 et respectivement un axe optique droit 15. L'axe optique gauche 13 et l'axe optique droit 15 appartiennent au plan horizontal 5.

L'appareil 1 présente une paroi interne 17 comprenant une zone temporale gauche 19 et une zone temporale droite 21 sensiblement en regard de la tempe gauche et respectivement de la tempe droite du patient.

La paroi présente également une zone centrale 22 ou champ nasal ménagée entre les fenêtres d'observation 12.

Les zones temporales 19, 21 et la zone centrale 22 sont pourvues de sources lumineuses ponctuelles 23 comprenant des diodes 25 électroluminescentes. La zone temporale gauche 19 est symétrique à la zone temporale droite 21 par rapport au plan vertical 7 et la zone centrale 22 est symétrique par rapport au plan vertical 7, la répartition des diodes 25 étant également symétrique.

Chaque diode 25 de la zone temporale gauche 19 présente par rapport au centre optique et à l'axe optique gauche 13 un angle d'incidence entre -30° et +30° par rapport à l'horizontale, le sens positif des angles étant défini vers le haut et un angle d'incidence entre +30° et +90° par rapport à la verticale, le sens positif des angles étant défini vers le champ visuel temporal de l'oeil gauche.

Ainsi, chaque diode 25 de la zone temporale droite 21 présente par rapport au centre optique et à l'axe optique droit 15 un angle d'incidence entre - 30° et +30° par rapport à l'horizontale, le sens positif des angles étant défini vers le haut et un angle d'incidence entre +30° et +90° par rapport à la verticale, le sens positif des angles étant défini vers le champ visuel temporal de l'oeil droit.

L'allumage et l'extinction des diodes 25 des zones temporales 19, 21 sont réalisés par des moyens de commandes externes non représentés qui comprennent une interface utilisateur permettant à l'utilisateur de définir l'ordre, la durée et l'intensité lumineuse de l'allumage des diodes 25.

L'appareil 1 d'exploration de la fonction visuelle est agencé pour effectuer une batterie d'examen de la vision du patient qui n'est pas détaillée dans ce texte, notamment par l'observation des yeux à travers les lucarnes 11, pour la vision de près, et les fenêtres 12, pour la vision intermédiaire et de loin.

L'appareil 1 d'exploration de la fonction visuelle comprend un dispositif 27 de détermination et/ou d'analyse du champ visuel du patient.

Dans un premier temps, nous allons détailler les éléments constituant le dispositif 27, puis dans un deuxième temps la réalisation de l'examen du champ visuel.

Le dispositif 27 comprend une partie mobile 29 sensiblement plane s'étendant selon un plan d'extension de la partie mobile.

La partie mobile 29 est pourvue de deux secteurs 31, 33 appartenant au plan d'extension, les secteurs étant symétriques par rapport au plan vertical 7. Chaque secteur 31, 33 comprend une pluralité de sources lumineuses ponctuelles sous forme de diodes 35 électroluminescentes montées sur ledit secteur 31, 33.

L'allumage et l'extinction des diodes 35 des secteurs 31, 33 sont réalisés par des moyens de commandes. Les moyens de commandes des diodes 35 des secteurs comprennent une carte électronique 37. La carte électronique 37 est montée sur la partie mobile 29. Selon un mode de réalisation préféré, les diodes 35 sont montées sur la carte électronique 37.

La carte électronique 37 est agencée pour revoir des commandes en provenance des moyens de commande externe. Ainsi l'ensemble des diodes 35 des secteurs 31, 33 et les diodes 25 des zones temporales 19, 21 sont agencées pour être commandées par un utilisateur utilisant l'interface utilisateur.

Chaque secteur 31, 33 comprend un repère visuel 39, 41 formé d'une ouverture rectangulaire débouchante à travers la partie mobile 29.

La partie mobile 29 se déplace entre une position escamotée et une position déployée. A la figure 1, la partie mobile 29 est dans la position escamotée. Le passage de la position escamotée à la position déployée se fait par translation selon un axe de repère 43, l'axe de repère 43 étant l'intersection du plan vertical 7 et du plan d'extension de la partie mobile.

Le déplacement de la position escamotée à la position déployée se fait dans une première direction 45 allant du haut vers le bas. Le déplacement de la position déployée à la position escamotée se fait dans une deuxième direction 47, inverse à la première direction 45.

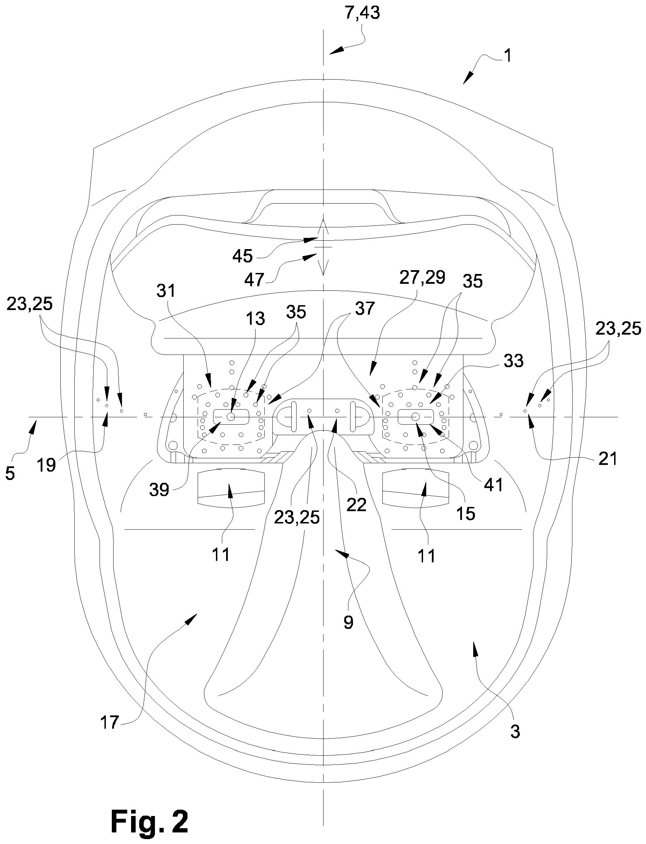

En position déployée, comme illustré à la figure 2, les secteurs 31, 33 sont en regard des yeux du patient, entre les yeux du patient et les fenêtres 12 d'observation. Les ouvertures débouchantes sont agencées de manière à ce que l'axe optique 13 gauche passe par le repère 39 gauche et l'axe optique droit 15 passe par le repère 41 droit.

Ainsi les sources lumineuses 23 des secteurs 31, 33 sont disposées de façon décalée par rapport aux axes optiques 13, 15 du patient.

Le plan d'extension de la partie mobile 29 est orthogonal au plan vertical 7 et au plan horizontal 5 et passe entre les yeux et la pointe du nez du patient.

Les diodes 35 du secteur gauche 31 sont réparties autour du repère gauche 39 afin que de présenter par rapport au centre optique et à l'axe optique 13 gauche des angles d'incidence inférieurs à 30°.

De même, du fait de la symétrie des secteurs 31, 33, les diodes 35 du secteur droit 33 sont réparties autour du repère droit 41 afin que de présenter par rapport au centre optique et à l'axe optique droit 15 des angles d'incidence inférieurs à 30°.

Après avoir présenté la partie mobile 29 et l'agencement de la partie mobile par rapport à l'appareil 1 d'exploration, nous allons détailler les autres éléments constituant le dispositif 27.

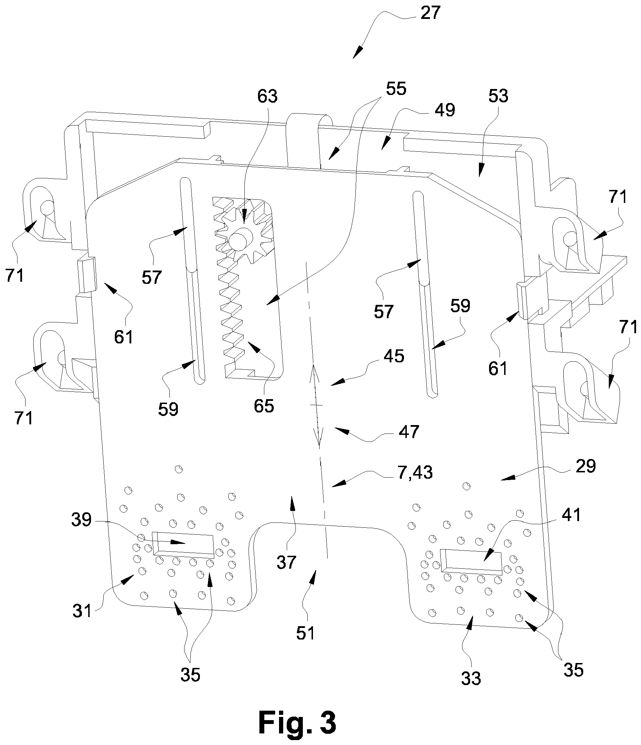

Comme illustré à la figure 3, le dispositif 27 comprend une partie fixe 49 et la partie mobile 29.

Une encoche 51 est située entre les secteurs de la partie mobile 29 et s'étend selon la deuxième direction 47. L'encoche 51 permet à la zone centrale 22 de rester dans le champ de vision du patient lorsque la partie mobile 29 est en position déployée.

La partie fixe 49 comprend un carter 53 pourvu d'une surface de guidage 55 de la partie mobile 29. Des moyens de guidage sont agencés sur la surface pour permettre un déplacement de la partie mobile 29 par rapport à la partie fixe 49 selon l'axe de référence 43.

Les moyens de guidage comprennent deux butées 57, chacune coopérant avec un sillon 59 dans la partie mobile 29, les positions extrêmes des sillons 59 par rapport aux butées 57 définissant les positions escamotée et déployée. Les moyens de guidage comprennent également deux ergots 61 de maintien de la partie mobile 29 contre la surface 55.

Le dispositif 27 comprend des moyens de déplacement de la partie mobile 29. Les moyens de déplacement de la partie mobile 29 comprennent un pignon 63 monté sur la partie fixe 49 et une crémaillère 65 prévue sur la partie mobile 29, le pignon 63 coopérant avec la crémaillère 65 de manière à ce que la rotation du pignon 63 provoque le déplacement de la partie mobile 29.

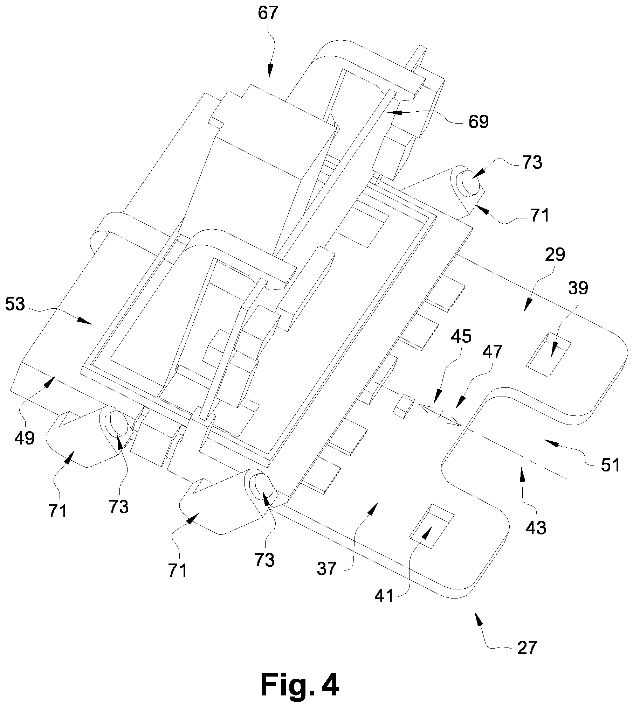

Comme illustré à la figure 4, la partie fixe 49 comprend un élément 67 d'entrainement et de commande de la rotation du pignon 63 situé du côté opposé de la partie mobile 29 par rapport à la surface 55.

L'élément 67 d'entrainement comprend un moteur électrique commandé par une carte électronique de commande 69 attenante au moteur. La carte électronique de commande 69 est connectée et contrôlée par les moyens de commande externe.

Le carter 53 de la partie fixe 49 comprend également des moyens de fixation du dispositif 27 à l'appareil 1 d'exploration. Les moyens de fixation comprennent quatre supports 71 destinés à recevoir des vis de fixation 73.

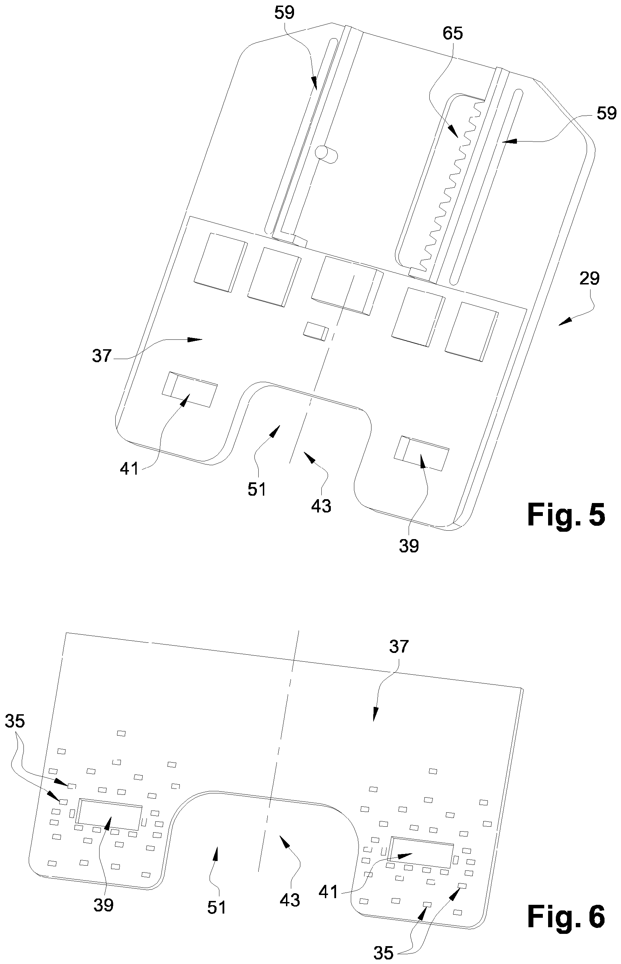

Comme illustré aux figures 5 et 6, les diodes 35 sont montées directement sur la carte électronique 37.

Après la description des différents éléments de l'appareil 1 d'exploration et du dispositif 27 le fonctionnement de l'appareil 1 est décrit ci-dessous.

Dans un premier temps, le patient positionne son visage dans l'emplacement 3 de positionnement de l'appareil 1. La partie mobile 29 peut indifféremment se trouver en position escamotée ou en position déployée.

Dans un deuxième temps, la partie mobile 29 est déplacée dans la position déployée si ce n'est pas déjà le cas.

Dans un troisième temps, le patient est invité à orienter les centres optiques de ses yeux selon les axes optiques 13, 15 à l'aide des repères visuels 39, 41.

Dans un quatrième temps, les diodes 35, 25 des secteurs et des zones temporales sont alternativement allumées et éteintes selon l'ordre donné par la commande utilisateur.

Selon un mode de réalisation préféré, une première série de diodes s'allume dans la zone temporale droite 21 et le secteur droit 33 pour examiner l'oeil droit et ensuite une deuxième série de diodes s'allume dans la zone temporale 19 gauche et le secteur gauche 31 pour examiner l'oeil gauche.

La commande peut être manuelle : dans ce cas l'utilisateur choisit lui-même l'ordre d'allumage et l'intensité de l'éclairage des diodes 25, 35. La commande peut être automatisée sous forme par exemple d'un allumage aléatoire des diodes.

Parallèlement à l'allumage des diodes, il est demandé au patient de signaler toute source lumineuse perçue.

Dans un cinquième temps, le champ visuel du patient est déduit à partir des sources lumineuses qu'il a perçues au cours de l'examen.

Comme il va de soi, l'invention ne se limite pas à la seule forme d'exécution de cette lunette escamotable, décrite ci-dessus à titre d'exemple, elle en embrasse au contraire toutes les variantes de réalisation.

## Revendications

1. Appareil (1) d'exploration de la fonction visuelle comprenant une partie présentant un emplacement de positionnement (3) apte à recevoir le visage d'un patient et un dispositif (27) de détermination et/ou d'analyse du champ visuel d'un patient, ledit dispositif (27) comprenant :
- une partie fixe (49) solidarisée à la partie de l'appareil (1) d'exploration de la fonction visuelle du patient présentant l'emplacement de positionnement (3),
- une partie mobile (29) pourvue de deux secteurs (31, 33) comprenant chacun une pluralité de sources lumineuses ponctuelles agencées pour s'allumer et s'éteindre,
la partie mobile étant déplaçable entre une position déployée dans laquelle chaque secteur est situé en regard d'un oeil correspondant du patient lorsque le visage du patient est disposé dans l'emplacement de positionnement, et une position escamotée, les secteurs (31, 33) étant symétriques par rapport à un plan vertical (7) passant par le segment de l'emplacement de positionnement (3) destiné à recevoir l'arête du nez du patient,
- des moyens de déplacement de la partie mobile (29) entre la position escamotée et la position déployée.

2. Appareil (1) d'exploration de la fonction visuelle selon la revendication 1, dans lequel les moyens de déplacement sont agencés pour déplacer en translation la partie mobile (29) entre ses positions escamotée et déployée.

3. Appareil (1) d'exploration de la fonction visuelle selon l'une des revendications 1 ou 2, dans lequel les moyens de déplacement de la partie mobile comprennent un pignon (63) monté sur la partie fixe (49) et une crémaillère (65) prévue sur la partie mobile (29), le pignon (63) coopérant avec la crémaillère (65) de manière à ce que la rotation du pignon (63) provoque le déplacement de la partie mobile (29).

4. Appareil (1) d'exploration de la fonction visuelle selon l'une des revendications 1 à 3, dans lequel la partie mobile (29) comprend des moyens de commande de l'allumage et de l'extinction des sources lumineuses des secteurs (31, 33).

5. Appareil (1) d'exploration de la fonction visuelle selon l'une des revendications 1 à 4, dans lequel chaque secteur (31, 33) comprend un repère visuel (39, 41) autour duquel sont réparties les sources lumineuses dudit secteur.

6. Appareil (1) d'exploration de la fonction visuelle selon la revendication 5, dans lequel chaque repère visuel (39, 41) est formé par une ouverture ménagée dans la partie mobile.

7. Appareil (1) d'exploration de la fonction visuelle selon la revendication 1, dans lequel les sources lumineuses de chaque secteur (31, 33) sont réparties autour du repère visuel (39, 41) dudit secteur (31, 33) de telle sorte que, en position déployée de la partie mobile, les sources lumineuses présentent par rapport au centre optique et à l'axe optique (13, 15) correspondant audit secteur (31, 33) des angles d'incidences inférieurs à 45°.

8. Appareil (1) selon l'une des revendications 1 ou 7, dans lequel le plan d'extension de la partie mobile est sensiblement vertical et est situé entre les yeux et la pointe du nez du patient lorsque le visage du patient est disposé dans l'emplacement de positionnement.

9. Appareil (1) selon l'une des revendications 1,7 ou 8, comprenant des sources lumineuses (23) supplémentaires montées sur une paroi interne (17) de l'appareil (1), afin que chaque source lumineuses supplémentaire (23) présente par rapport au centre optique et à l'axe optique (13, 15) de l'oeil le plus proche de ladite source (23) :
- un angle d'incidence entre -30° et +30° par rapport à l'horizontale, le sens positif des angles étant défini vers le haut et
- un angle d'incidence entre +30° et +90° par rapport à la verticale, le sens positif des angles étant défini vers le champ visuel temporal de l'oeil le plus proche.

10. Appareil (1) selon l'une des revendications 1, 7, 8 ou 9, dans lequel les sources lumineuses des secteurs et les sources lumineuses supplémentaires comprennent des diodes électroluminescentes.

## Patentansprüche

1. Gerät (1) zum Erforschen der Sehfunktion, das einen Teil umfasst, der eine Positionierungsstelle (3) aufweist, die geeignet ist, um das Gesicht eines Patienten aufzunehmen, und eine Vorrichtung (27) zum Bestimmen und/oder zur Analyse des Blickfelds eines Patienten, wobei die Vorrichtung (27) Folgendes umfasst:
- einen stationären Teil (49), der fest mit dem Teil des Geräts (1) zum Erforschen der Sehfunktion des Patienten, der die Positionierungsstelle (3) aufweist, verbunden ist,
- einen beweglichen Teil (29), der mit zwei Sektoren (31, 33) versehen ist, die jeweils eine Vielzahl punktförmiger Lichtquellen umfassen, die eingerichtet sind, um sich ein- und auszuschalten,
wobei der bewegliche Teil zwischen einer ausgefahrenen Position, in der jeder Sektor gegenüber einem entsprechenden Auge des Patienten liegt, wenn das Gesicht des Patienten in der Positionierungsstelle angeordnet ist, und einer eingefahrenen Position bewegbar ist, wobei die Sektoren (31, 33) in Bezug auf eine vertikale Ebene (7), die durch das Segment der Positionierungsstelle (3) verläuft, die dazu bestimmt ist, den Rücken der Nase des Patienten aufzunehmen, symmetrisch sind,
- Mittel zum Bewegen des beweglichen Teils (29) zwischen der eingefahrenen Position und der ausgefahrenen Position.

2. Gerät (1) zum Erforschen der Sichtfunktion nach Anspruch 1, wobei die Mittel zum Bewegen eingerichtet sind, um den beweglichen Teil (29) zwischen seiner eingefahrenen und ausgefahrenen Position zu bewegen.

3. Gerät (1) zum Erforschen der Sichtfunktion nach einem der Ansprüche 1 oder 2, wobei die Mittel zum Bewegen des beweglichen Teils ein Ritzel (63) umfassen, das auf den stationären Teil (49) montiert ist, und eine Zahnstange (65), die auf dem beweglichen Teil (29) vorgesehen ist, wobei das Ritzel (63) mit der Zahnstange (65) derart zusammenwirkt, dass die Drehung des Ritzels (63) die Bewegung des beweglichen Teils (29) hervorruft.

4. Gerät (1) zum Erforschen der Sichtfunktion nach einem der Ansprüche 1 bis 3, wobei der bewegliche Teil (29) Mittel zum Steuern des Einschaltens und Ausschaltens der Lichtquellen der Sektoren (31, 33) umfasst.

5. Gerät (1) zum Erforschen der Sichtfunktion nach einem der Ansprüche 1 bis 4, wobei jeder Sektor (31, 33) eine visuelle Kennzeichnung (39, 41) umfasst, um die die Lichtquellen des Sektors verteilt sind.

6. Gerät (1) zum Erforschen der Sichtfunktion nach Anspruch 5, wobei jede visuelle Kennzeichnung (39, 41) von einer Öffnung, die in dem beweglichen Teil eingerichtet ist, gebildet ist.

7. Gerät (1) zum Erforschen der Sichtfunktion nach Anspruch 1, wobei die Lichtquellen jedes Sektors (31, 33) um die sichtbare Kennzeichnung (39, 41) des Sektors (31, 33) derart verteilt sind, dass die Lichtquellen in ausgefahrener Position des beweglichen Teils in Bezug auf die optische Mitte und die optische Achse (13, 15), die dem Sektor (31, 33) entsprechen, Neigungswinkel kleiner als 45° aufweisen.

8. Gerät (1) nach einem der Ansprüche 1 oder 7, wobei die Erweiterungsebene des beweglichen Teils im Wesentlichen vertikal ist und zwischen den Augen und der Spitze der Nase des Patienten liegt, wenn das Gesicht des Patienten in der Positionierungsstelle angeordnet ist.

9. Gerät (1) nach einem der Ansprüche 1, 7 oder 8, das zusätzliche Lichtquellen (23) umfasst, die auf einer internen Wand (17) des Geräts (1) montiert sind, damit jede zusätzliche Lichtquelle (23) in Bezug auf die optische Mitte und auf die optische Achse (13, 15) des Auges, das der Quelle (23) am nächsten liegt, Folgendes aufweist:
- einen Einfallswinkel zwischen -30° und +30° in Bezug auf die Waagerechte, wobei die positive Richtung der Winkel nach oben definiert ist, und
- einen Einfallswinkel zwischen +30° und +90° in Bezug auf die Senkrechte, wobei die positive Richtung der Winkel zu dem Schläfenblickfeld des am nächsten liegenden Auges definiert ist.

10. Gerät (1) nach einem der Ansprüche 1, 7, 8 oder 9, wobei die Lichtquellen der Sektoren und die zusätzlichen Lichtquellen Licht emittierende Dioden umfassen.

## Claims

1. An apparatus (1) for examining the visual function comprising a portion having a positioning location (3) capable of receiving the face of a patient and a device (27) for determining and/or analyzing the visual field of a patient, said device (27) comprising:
- a fixed portion (49) secured to the portion of the apparatus (1) for examining the patient visual function which has the positioning location (3),
- a movable portion (29) provided with two sectors (31, 33) each comprising a plurality of point light sources arranged to be turned on and off,
the movable portion being displaceable between a deployed position in which each sector is located facing a corresponding eye of the patient when the patient's face is disposed in the positioning location, and a retracted position, the sectors (31, 33) being symmetrical relative to a vertical plane (7) passing through the segment of the positioning location (3) intended to receive the bridge of the patient's nose,
- means for displacing the movable portion (29) between the retracted position and the deployed position.

2. The visual function examining apparatus (1) according to claim 1, wherein the displacement means are arranged to displace in translation the movable portion (29) between its retracted and deployed positions.

3. The visual function examining apparatus (1) according to any of claims 1 or 2, wherein the means for displacing the movable portion comprise a pinion (63) mounted on the fixed portion (49) and a rack (65) provided on the movable portion (29), the pinion (63) cooperating with the rack (65) so that the rotation of the pinion (63) causes the displacement of the movable portion (29).

4. The visual function examining apparatus (1) according to any of claims 1 to 3, wherein the movable portion (29) comprises means for controlling the switching on and off of the light sources of the sectors (31, 33).

5. The visual function examining apparatus (1) according to any of claims 1 to 4, wherein each sector (31, 33) comprises a visual cue (39, 41) about which the light sources of said sector are distributed.

6. The visual function examining apparatus (1) according to claim 5, wherein each visual cue (39, 41) is formed by an opening provided in the movable portion.

7. The visual function examining apparatus (1) according to claim 1, wherein the light sources of each sector (31, 33) are distributed about the visual cue (39, 41) of said sector (31, 33) such that, in the deployed position of the movable portion, the light sources have, relative to the optical center and to the optical axis (13, 15) corresponding to said sector (31, 33), angles of incidence less than 45°.

8. The visual function examining apparatus (1) according to any of claims 1 or 7, wherein the plane of extension of the movable portion is substantially vertical and is located between the eyes and the tip of the patient's nose when the patient's face is disposed in the positioning location.

9. The visual function examining apparatus (1) according to any of claims 1, 7 or 8, comprising additional light sources (23) mounted on an inner wall (17) of the apparatus (1), so that each additional light source (23) has, relative to the optical center and to the optical axis (13, 15) of the eye closest to said source (23):
- an angle of incidence between -30° and +30° relative to the horizontal, the positive direction of the angles being defined upwards, and
- an angle of incidence between +30° and +90° relative to the vertical, the positive direction of the angles being defined towards the temporal visual field of the nearest eye.

10. The visual function examining apparatus (1) according to any of claims 1, 7, 8 or 9, wherein the light sources of the sectors and the additional light sources comprise light-emitting diode.
